(19) Europäisches Patentamt
European Patent Office
Office européen des brevets

(11) Numéro de publication: **0 185 020**
**B1**

(12) # FASCICULE DE BREVET EUROPEEN

(45) Date de publication du fascicule du brevet: **17.02.88**

(51) Int. Cl.⁴: **C 07 C 149/437**, C 07 C 153/07, C 07 C 147/02, C 07 C 147/14, A 61 K 31/17, A 61 K 31/215

(21) Numéro de dépôt: **85901474.8**

(22) Date de dépôt: **10.04.85**

(86) Numéro de dépôt international: **PCT/FR 85/00080**

(87) Numéro de publication internationale: **WO 85/04655 (24.10.85** Gazette **85/23)**

(54) **COMPOSES NITROSOUREES, LEUR PREPARATION ET LEUR UTILISATION EN CHIMIOTHERAPIE ANTICANCEREUSE.**

(30) Priorité: **11.04.84 FR 8405733**

(43) Date de publication de la demande: **25.06.86 Bulletin 86/26**

(45) Mention de la délivrance du brevet: **17.02.88 Bulletin 88/7**

(84) Etats contractants désignés: **AT BE CH DE FR GB IT LI LU NL SE**

(56) Documents cités: **EP - A - 0 021 991**

(73) Titulaire: **CENTRE NATIONAL DE LA RECHERCHE SCIENTIFIQUE (CNRS), 13 Quai Anatole France, F-75700 Paris (FR)**
Titulaire: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **GODENECHE, Denise, 84, avenue Thermale, F-63400 Chamalières (FR)**
Inventeur: **IMBACH, Jean-Louis, "Clos des Oliviers" 1108, rue de Las Sorbes, F-34000 Montpellier (FR)**
Inventeur: **MADELMONT, Jean-Claude, 32, boulevard du Chauffour, F-63540 Romagnat (FR)**
Inventeur: **MEYNIEL, Gaston, 77, avenue Raymond Bergougnan, F-63100 Clerrint (FR)**
Inventeur: **MOREAU, Marie-France, Impasse Voltaire, F-63540 Romagnat (FR)**
Inventeur: **OIRY, Joel, 31, lotissement Château Bon, Route de Lavérune F-34100 Montpellier (FR)**
Inventeur: **PARRY, Daniel, 11bis, rue de Ceyrat, F-63110 Beaumont (FR)**

(74) Mandataire: **Warcoin, Jacques et al, Cabinet Régimbeau 26, avenue Kléber, F-75116 Paris (FR)**

**EP 0 185 020 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne, de façon générale, de nouveaux composés nitroso-urées, des procédés pour leur préparation ainsi que des compositions pharmaceutiques à base de tels composés, utiles notamment en chimiothérapie anticancéreuse.

Plus particulièrement, la présente invention concerne des composés à activité oncostatique dérivés de la cystéamine appartenant à la classe des chloroéthyl nitroso-urées.

Les chloroéthyl nitroso-urées constituent une classe de composés utilisés en chimiothérapie anticancéreuse dans le traitement des glioblastomes de la maladie de Hodgkin et de cancers gastro-intestinaux.

Les plus utilisées d'entre elles sont la BCNU, la CCNU et la méthyl CCNU. Parmi les nombreux dérivés de cette série préparés récemment dans le monde, quelques-uns ont été retenus sur la base de la largeur de leur spectre d'action sur les tumeurs murines pour les essais cliniques phase I et II. Il s'agit de:

la chlorozotocine, l'ACNU, l'HECNU, la RFCNU, la CNCC.

Dans cette série, la CNCC semble être la substance la plus prometteuse, comme l'indique le tableau 1.

En effet, à l'issue des essais dont les résultats sont rassemblés dans ce tableau, il apparaît que le CNCC possède une activité tant qualitative que quantitative très intéressante. Elle se montre active à l'égard de tous les types de tumeur répertoriés dans le tableau I, excepté sur le fibrosarcome I.C.I.G. Cette activité est quantitativement plus importante que celles observées avec les autres chloroéthyl nitroso-urées.

*Tableau I*

*Augmentation de la durée de survie (I.L.S.: T/C×100)*[1]
(traitement par voie I.P. au jour 1,5,9)

| TUMEUR | CHLOROZOTOCINE 15 mg/kg I.P. | RPCNU 15 mg/kg I.P. | RFCNU 20 mg/kg I.P. | HECNU 10 mg/kg I.P. | CNCC 30 mg/kg I.P. |
|---|---|---|---|---|---|
| Leucémie L 1210 (greffée par voie i.c.) | N.S. | N.S. | 167 | ∞ | ∞ |
| Carcinome pulmonaire de Lewis (implanté en sous-cutané) | N.S. | N.S. | N.S. | ∞ | ∞ |
| Mélano-carcinome B 16 (implanté en sous-cutané) | N.S. | 151 | 144 | 198 | 200 |
| Gliome 26 (implanté en sous-cutané) | N.S. | 127 | N.S. | 182 | 200 |
| Adénocarcinome mammaire TM2 (implanté en sous-cutané) | N.S. | 134 | N.S. | 165 | 185 |
| Fibrosarcome I.C.I.G. $C_1$ (implanté en sous-cutané) | ∞ | | ∞ | N.S. | N.S. |
| Carcinome du côlon (côlon 26) (implanté en sous-cutané) | ∞ | N.S. | N.S. | ∞ | ∞ |
| M 555 ovarienne (implanté en sous-cutané) | ∞ | ∞ | N.S. | 210 | > 254 |

N.S. = Non significatif; ∞ = 50% en plus des animaux traités sont guéris.
[1] Reproduit de «Nitrosoureas in Cancer Treatment», Ed. B. Serrou, P.S. Schein and J.L. Imbach, Elsevier North Holland, 1981, 132.

Enfin, on remarque que la CNCC est employée à des doses supérieures (30 mg/kg) à celles avec lesquelles on utilise la chlorozotocine, la RPCNU, la RFCNU ou la HECNU, ce qui est un atout supplémentaire en chimiothérapie anticancéreuse. Dans ce domaine, on connaît en effet la nécessité de trouver un compromis entre une dose efficace de produit, permettant d'obtenir la régression maximale de la tumeur, et un minimum d'effets secondaires néfastes.

La CNCC possède donc un spectre d'action étendu et permet un traitement des tumeurs murines plus efficace.

C'est pourquoi la présente invention concerne de nouveaux agents cytostatiques dérivés de la CNCC, présentant une activité améliorée par rapport aux produits de la technique antérieure et/ou un spectre d'activité différent.

Il s'agit des composés de formule générale I:

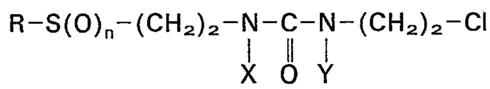

dans laquelle:

- X et Y sont définis individuellement par H ou $-NO$, l'un au moins étant un groupement $-NO$,
- n est égal à 0, 1 ou 2,
- R est l'hydrogène, un radical aliphatique, cycloaliphatique, cycloaliphatique-alkyl, aryle ou aralcoyle, ou bien encore un groupement

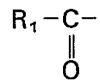

dans lequel $R_1$ est un radical alkyle inférieur ou aryle,

l'ensemble de ces radicaux étant éventuellement substitué.

Par radical aliphatique, on entend des radicaux alkyles, alcényles ou alcynyles, droits ou ramifiés, comportant de 1 à 8 atomes de carbone. Il s'agit de préférence de radicaux alkyles inférieurs, c'est-à-dire comportant de 1 à 7 atomes de carbone, et plus particulièrement des radicaux en $C_1$ à $C_3$ tels que méthyle, éthyle.

Par cycloaliphatique, on entend des radicaux comportant 3 à 8 atomes de carbone, saturés ou non. En particulier, on préfère le radical cyclohexyle.

Par aryle, on entend des radicaux en $C_6$-$C_{30}$ renfermant un ou plusieurs noyaux, du type benzénique par exemple, substitués ou non. Parmi ces radicaux, on préfère en particulier le radical phényle, éventuellement substitué par un groupement $-NO_2$, par exemple le groupement:

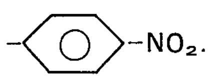

Par cycloaliphatique-alkyl, on entend un radical alkyle inférieur substitué par un ou plusieurs radicaux cycloaliphatiques. De même par aralcoyle, on entend un radical alkyle inférieur substitué par un ou plusieurs radicaux aryles. Les termes «alkyle inférieur», «cycloaliphatique» et «aryle» sont employés comme définis précédemment. Parmi ces radicaux, on préfère notamment le radical trityle ($(C_6H_5)_3-C-$).

Parmi les composés selon l'invention, on préfère en particulier les dérivés de formule II:

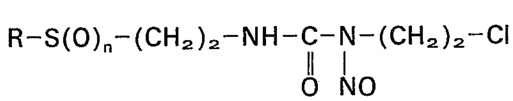

pour lesquels le groupement $-NO$ est porté par l'azote côté chlore.

Parmi les composés selon l'invention les plus intéressants, il faut citer:

• 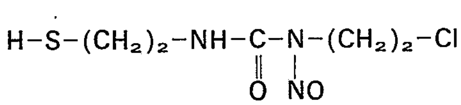

N-[N-(chloro-2 éthyl)N-nitrosocarbamoyl]-cystéamine.

• 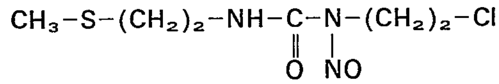

S-méthyl N-[N-(chloro-2 éthyl)N-nitrosocarbamoyl]cystéamine.

• 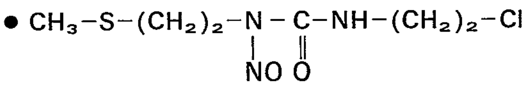

S-méthyl N-nitroso-N-[N-(chloro-2 éthyl)carbamoyl]cystéamine.

• 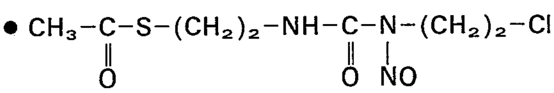

S-acétyl N-[N-(chloro-2 éthyl)N-nitrosocarbamoyl]cystéamine.

• 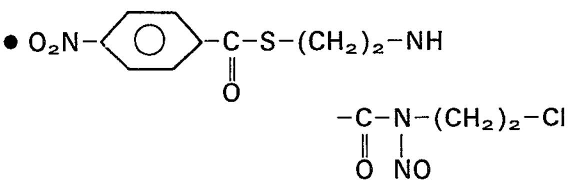

S-para-nitrobenzoyl N-[N-(chloro-2 éthyl)N-nitrosocarbamoyl]cystéamine.

• 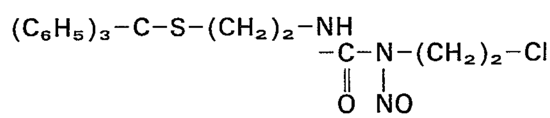

S-trityl-N-[N-(chloro-2 éthyl)N-nitrosocarbamoyl]cystéamine.

La présente invention concerne également les sulfoxydes de formule:

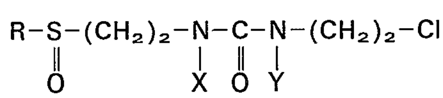

ainsi que les sulfones de formule:

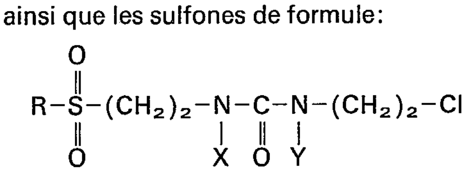

correspondant à l'oxydation ménagée ou complète du soufre des composés dérivés de la cystéamine de formule:

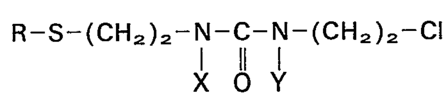

De plus, l'invention a pour objet des mélanges isomériques des composés de formule générale I:

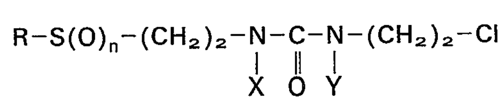

compte tenu du fait que lorsque X et Y ont des significations différentes — lorsque l'un est l'hydrogène, l'autre le groupement $-NO-$, suivant que $-NO$ est porté par l'azote côté chlore ou côté soufre, on obtient deux isomères de position.

En particulier, l'invention concerne le mélange des isomères de position suivant:

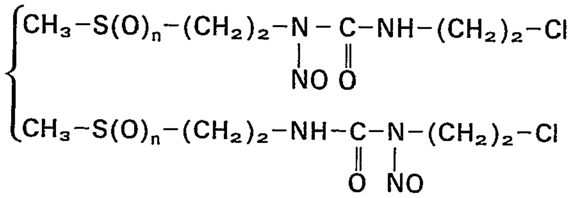

Les composés selon la présente invention peuvent être préparés par différents procédés.

Pour les composés dans lesquels R est un hydrogène, un radical aliphatique, cycloaliphatique, cycloaliphatique-alkyl, aryle ou aralcoyle et n = 0 par nitrosation du composé correspondant de formule:

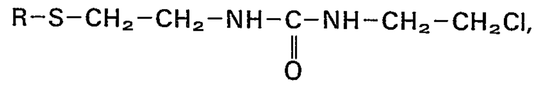

par action d'un nitrite de métal alcalin ou alcalino-terreux en présence d'un acide organique, en particulier par action de $NaNO_2$ dans l'acide formique.

En particulier, le *schéma I* illustre un procédé de préparation des composés de formule:

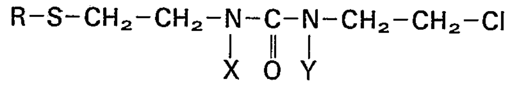

dans lequel R est $-CH_3$.

SCHÉMA I

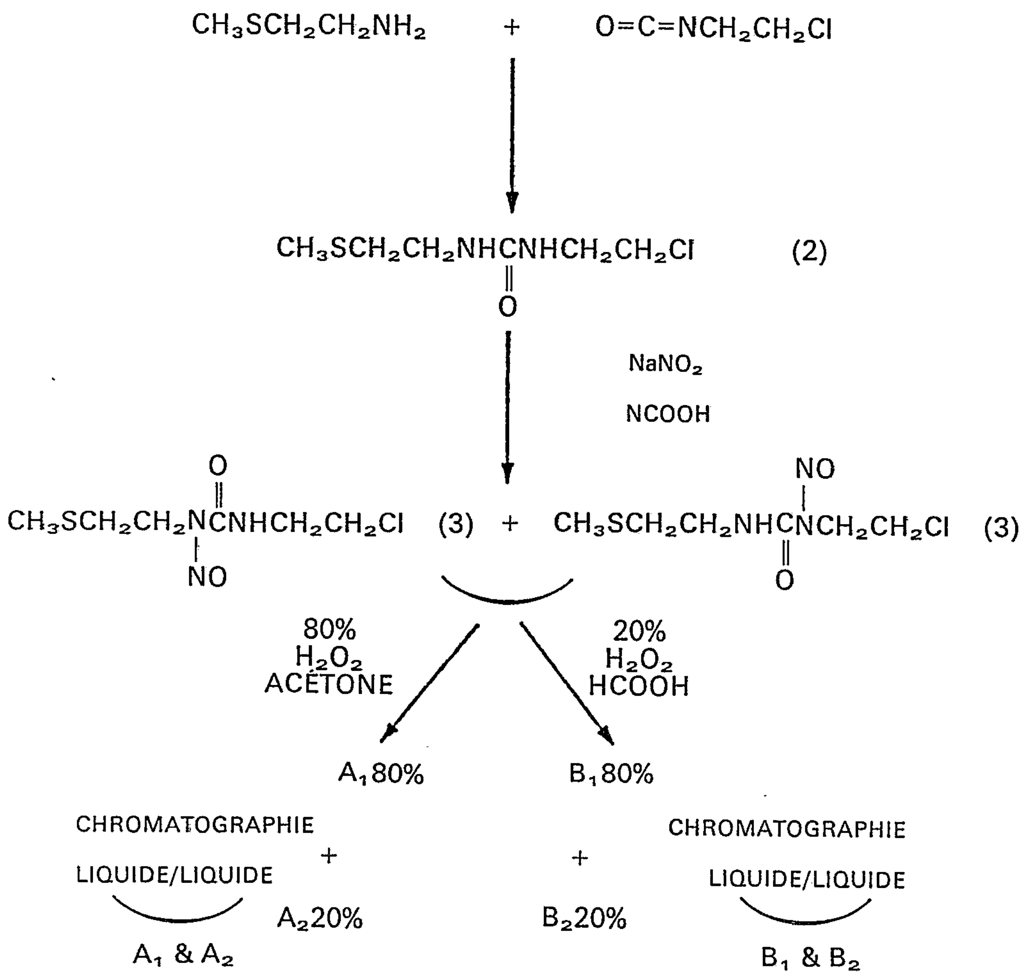


On prépare l'urée (2) en faisant réagir la S-méthyl-cystéamine avec du chloroéthyl-isocyanate. On effectue ensuite la nitrosation de l'urée (2) obtenue par $NaNO_2$ en présence d'acide formique. On obtient alors un mélange constitué de deux isomères de position suivants: la S-méthyl N-[N-(chloro-2 éthyl) N-nitrosocarbamoyl]cystéamine (3) et la S-méthyl N-nitroso[N-(chloro-2 éthyl) N-carbamoyl] cystéamine (4).

L'analyse du spectre RMN de ce mélange révèle la présence de ces deux isomères, l'un nitrosé côté chlore (3), l'autre nitrosé côté soufre (4) dans la proportion de 25%/75%.

La séparation de ces deux isomères étant très

délicate, on préférera utiliser pour obtenir un produit pur le procédé suivant qui consiste à faire réagir:

$H_2N-CH_2-CH_2Cl$ sur

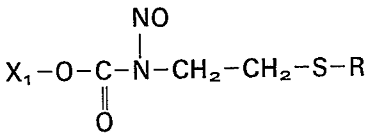

dans lequel $X_1$ est un groupe activant pour obtenir le dérivé nitrosé côté soufre ou

$R-S-Ch_2-Ch_2-NH_2$ sur

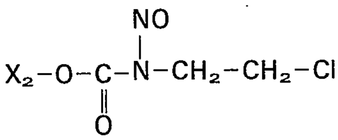

dans lequel $X_2$ est un groupe activant pour obtenir le dérivé nitrosé côté chlore.

On entend par groupe activant $X_1$ ou $X_2$ tout groupe fonctionnel favorisant l'addition du réactif nitrosé, tel qu'un nitrosocarbamate, sur la fonction amine primaire des éthylamines:

$Cl-CH_2-CH_2-NH_2$
ou $R-S-CH_2-CH_2-NH_2$

de façon à préparer les composés chloroéthyl-nitroso-urées selon l'invention, respectivement nitrosés côté soufre ou côté chlore. Le brevet français N° 2 487 343 mentionne les groupes activants classiquement utilisés, à cet effet; on cite en particulier le p-nitrophényle

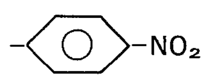

Les schémas II et III illustrent des procédés de préparation des composés selon l'invention, purs et non pas en mélange.

— Le *schéma II* a pour objet un procédé de préparation de la S-méthyl-N-nitroso-[N-(chloro-2 éthyl)N-carbamoyl] cystéamine (4). Ce composé (4) pur est obtenu par addition sur la chloro-2 éthylamine $H_2NCH_2CH_2Cl$ du nitrosocarbamate (10) dont l'extrémité

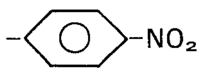

constitue le groupe $X_1$ activant permettant d'obtenir le dérivé chloroéthylnitroso-urée (4) nitrosé côté soufre.

— Le *schéma III* a pour objet un procédé de préparation de l'isomère de position du composé précédent (4), c'est-à-dire la S-méthyl-N-[N-(chloro-2 éthyl)N-nitrosocarbamoyl] cystéamine (3). Pour ce faire, on effectue l'addition du nitrosocarbamate (11) sur la S-méthylcystéamine, l'extrémité

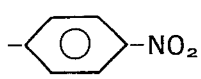

du nitrosocarbamate correspondant dans ce cas à un groupe activant $X_2$ permettant d'obtenir le dérivé chloroéthyl-nitroso-urée (3) nitrosé côté chlore.

SCHEMA II

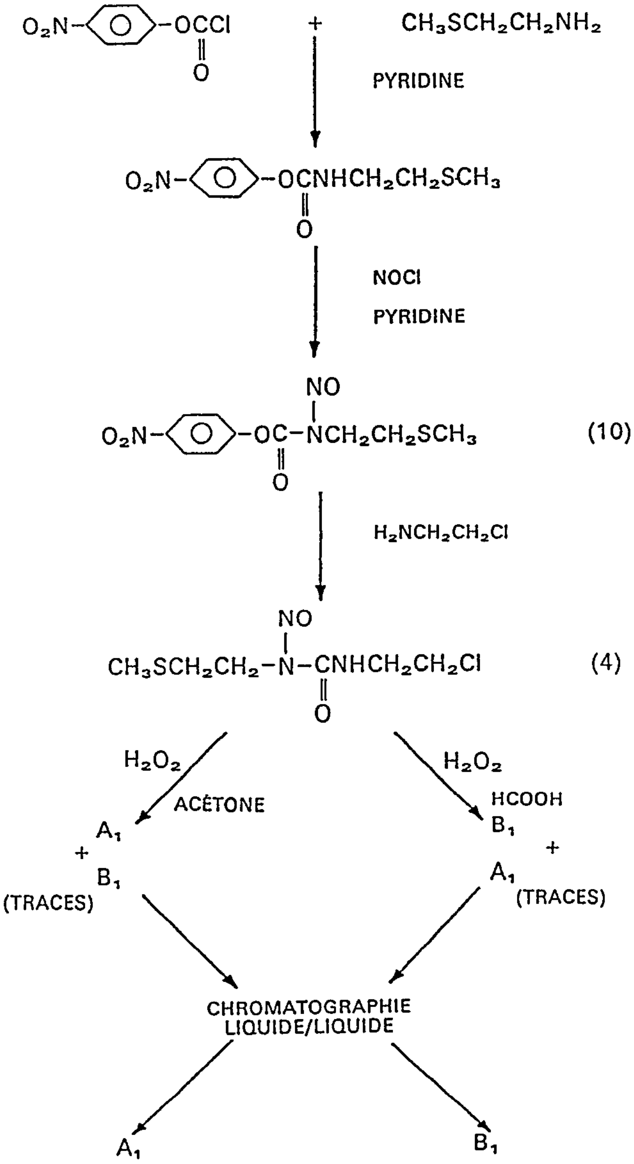


Pour obtenir un composé dans lequel R est

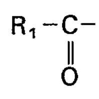 et n = 0,

on effectuera de préférence la thioestérification d'un composé de formule:

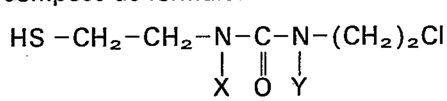

par un dérivé réactif de l'acide correspondant

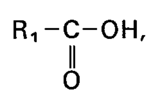

par exemple le chlorure d'acide.

SCHÉMA III

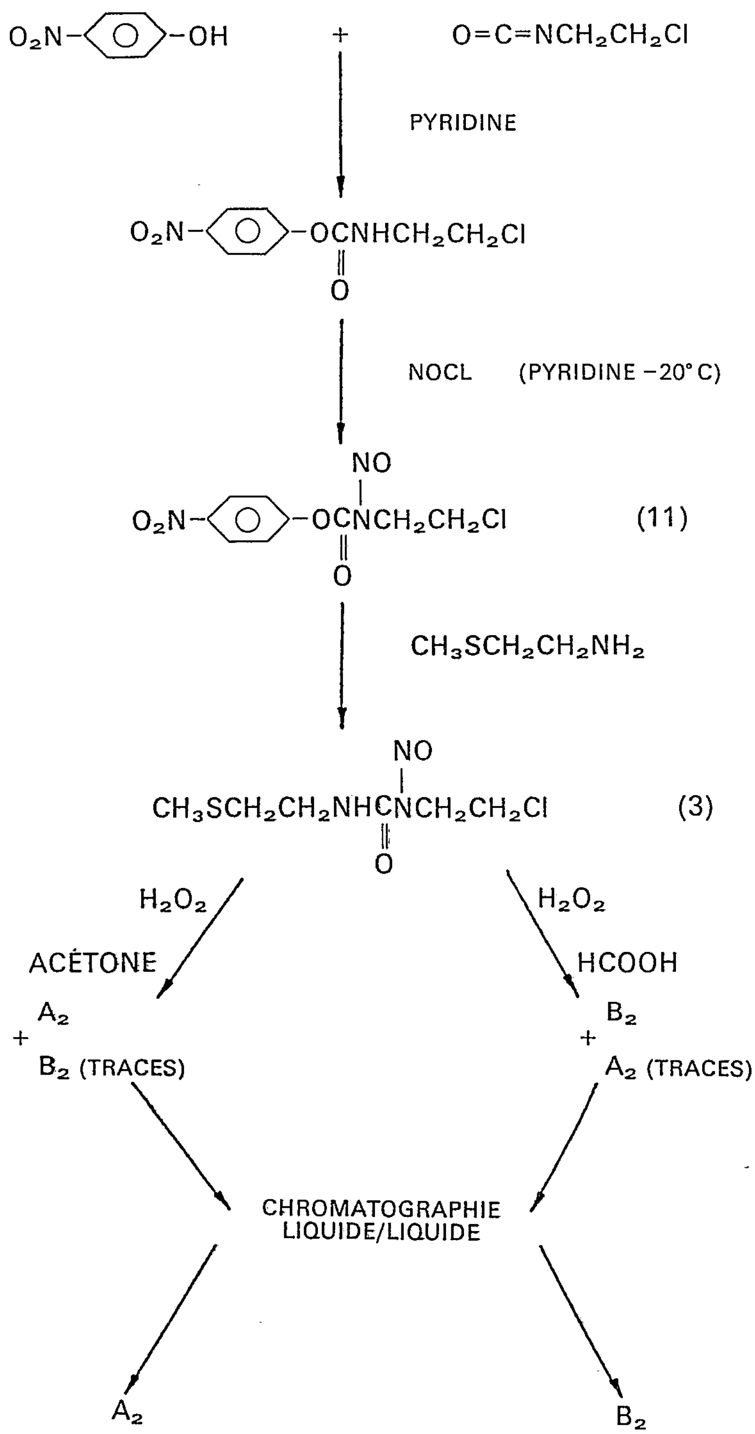


Cette thioestérification sera effectuée dans les conditions habituellement mises en œuvre par l'homme de l'art, par exemple en présence d'un solvant anhydre, tel que la pyridine. Elle est illustrée par la dernière étape du schéma II.

Le *schéma IV* concerne un procédé de préparation de la N-[N-(chloro-2 éthyl) N-nitrosocarbamoyl] cystéamine (1) par addition de N-(chloro-2 éthyl) N-nitrosocarbamoyl cystéamine (1) par addition de N-(chloro-2 éthyl) N-nitrosocarbamate de para-nitrophényl (6) sur la cystéamine (7). Ainsi que précédemment, le réactif (6) constitue le composé porteur d'un groupe activant

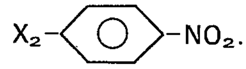

permettant d'obtenir un dérivé nitrosé côté chlore. Toutefois, cette réaction devra être conduite sous courant gazeux inerte azote par exemple, car le thiol (1) tend à se dimériser pour former la CNCC.

La dernière étape du schéma IV correspond à la préparation des composés de formule générale I

dans laquelle n = 0 et R est 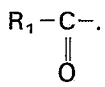

Le composé chloroéthylnitroso-urée (1) obtenu est alors soumis à l'action du chlorure d'acide

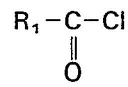

correspondant au produit final (12) que l'on désire préparer.

SCHÉMA IV

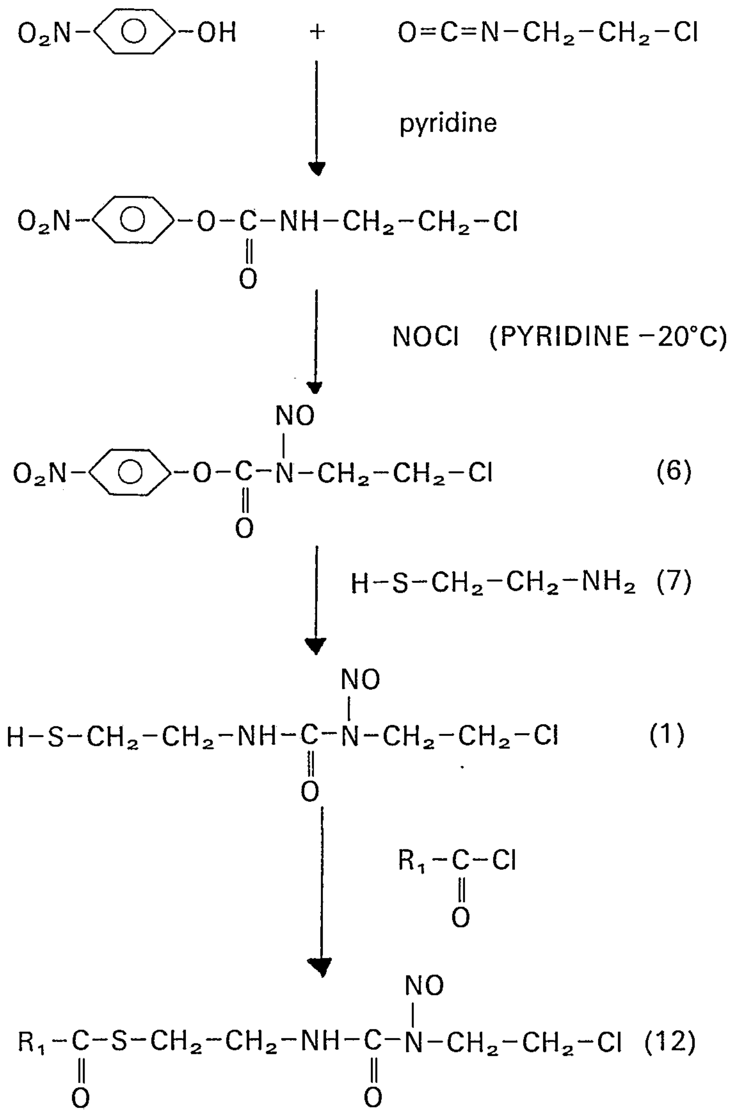


Les composés oxydés selon l'invention: sulfoxydes (n = 1) et sulfones (n = 2), peuvent être préparés par oxydation ménagée ou complète du soufre des composés correspondants (n = 0) avec un peroxyde tel que le peroxyde d'hydrogène.

Pour obtenir les sulfoxydes selon l'invention, de formule:

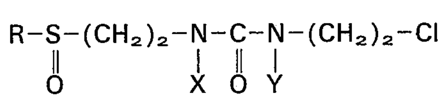

on soumet les composés correspondants de formule:

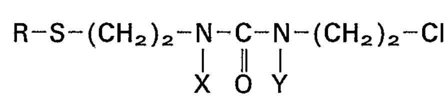

à l'action d'un peroxyde tel que le peroxyde d'hydrogène, en présence d'acétone, sous agitation.

Pour obtenir les sulfones selon l'invention, de formule:

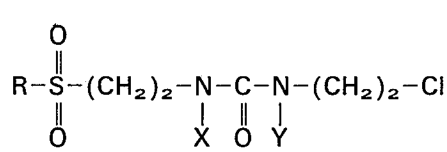

on soumet de même les composés correspondants de formule:

$$R-S-(CH_2)_2-\underset{\underset{X}{|}}{N}-\underset{\underset{O}{\|}}{C}-\underset{\underset{Y}{|}}{N}-(CH_2)_2-Cl$$

à l'action d'un peroxyde, tel que le peroxyde d'hydrogène, en présence d'acide formique, sous agitation.

La synthèse des composés selon l'invention sous forme de sulfoxydes et de sulfones est représentée par les dernières étapes des schémas I, II et III.

Par souci de simplicité, on emploiera par la suite la convention suivante:

- la lettre A désigne les composés selon l'invention sous forme de sulfoxydes et la lettre B sous forme de sulfones,
- l'indice 1 se rapporte aux composés nitrosés côté soufre, en particulier la S-méthyl-N-nitroso[N-(chloro-2 éthyl) N-carbamoyl] cystéamine, et l'indice 2 aux composés nitrosés côté chlore, en particulier la S-méthyl N-[N-(chloro-2 éthyl) N-nitrosocarbamoyl] cystéamine.

Les sulfoxydes ($A_1$ et $A_2$) et les sulfones ($B_1$ et $B_2$) obtenus, ainsi que les mélanges de ceux-ci, sont purifiés de façon classique par chromatographie, notamment par chromatographie liquide/liquide.

En outre, les composés selon l'invention présentant une activité oncostatique, un autre objet de la présente invention concerne des compositions pharmaceutiques comportant à titre de principe actif au moins un desdits composés.

De telles compositions seront avantageusement utilisées en chimiothérapie anticancéreuse.

Compte tenu du large spectre d'action des composés selon l'invention, les compositions pharmaceutiques les contenant pourront être utilisées pour le traitement de tumeurs circulantes telles que les leucémies, ou de tumeurs solides telles que: mélanocarcinome, carcinome pulmonaire, carcinome du côlon, adénocarcinome mammaire, gliome, fibrosarcome par exemple.

De telles compositions pourront être administrées par voie parentérale telle que par voie intraveineuse, ou bien par voie orale.

Elles pourront se présenter sous forme de comprimés, de capsules, de solutions ou suspensions dans des milieux aqueux ou organiques non toxiques, sous forme de poudres dispersables ou de solutions ou suspensions dans des milieux aqueux ou organiques non toxiques injectables.

Enfin, ces compositions seront administrées à des doses comprises entre 10 mg et 100 mg de principe actif/jour. Pour chaque traitement, la dose sera déterminée en fonction du type de tumeur, de l'état du patient, de la toxicité du produit, entre autres.

Les exemples ci-après permettront de mettre en évidence d'autres caractéristiques et avantages de la présente invention.

*Exemple 1:*

*Préparation du mélange S-méthyl N-[N-(chloro-2 éthyl)N-nitrosocarbamoyl)] cystéamine et de la S-méthyl N-[N-(chloro-2 éthyl)N-carbamoyl)] N-nitrosocystéamine* (schéma I)

A — a) *Préparation de la S-méthyl-N-[N-(chloro-2 éthyl)N-carbamoyl] cystéamine:*

Un mélange équimolaire de 50 mM de S-méthylcystéamine et de chloro-éthyl-isocyanate dans 100 cm³ d'éther anhydre est agité pendant 12 h à température ambiante.

Après 1 h d'agitation la solution limpide se trouble. Après 12 h l'abondant précipité blanc est filtré et rincé par l'éther. Le produit obtenu peut être utilisé tel quel pour les réactions suivantes:

Rdt: 80%

F: 74°C

CCM: 5% EtOH $CHCl_3$ Silice Merck S1 60 F 254

Rf: 0,5

IR: (KBr) $\nu \cdot cm^{-1}$: 3320 (NH), 2900 à 3000 (CH), 1630 (C) 1570, 1520 $(NH-\underset{\underset{O}{\|}}{C}=O)$

RMN ($CDCl_3$) δ ppm [2,06 s (3) $\underline{CH_3}$ S], [2,60 t (2) − S $\underline{CH_2}CH_2$] [3,36 m (2) − S $CH_2\underline{CH_2}$], [3,4 à 3,6 m (4) NH $\underline{CH_2CH_2}$Cl] [5,23 à 5,9 m (2) $\underline{NH}$]

b) *Nitrosation de la S-méthyl-N-[N-(chloro-2 éthyl)N-carbamoyl] cystéamine:*

40 mM de l'urée obtenue en a) sont solubilisées dans 100 cm³ d'un mélange acide formique-eau volume à volume (70/30). A la solution refroidie à 0°C, on ajoute par petites portions en 1 h 400 mM de nitrite de sodium en contrôlant la température. La solution est agitée 1 h à 0°C après la fin de l'addition de $NaNO_2$.

On dilue le milieu réactionnel par 100 cm³ d'eau glacée puis extrait par le chloroforme (5 × 50 cm³), sèche rapidement puis concentre sous pression réduite.

Le produit liquide peut soit être utilisé tel quel soit purifié des substances de dégradation (peu abondantes 3 à 4%) par chromatographie liquideliquide sur colonne de silice: éluant $CH_2Cl_2$.

Le dosage des deux isomères effectué en HPLC donne les proportions 80/20, dérivé NO côté chlore/dérivé NO côté chlore.

Rdt: 90%.

B — *Ce mélange peut également être préparé selon le procédé ci-après:*

a) *S-Méthyl N-[N-(chloro-2 éthyl-N-carbamoyl] cystéamine* (schéma I):

Une solution de 1,82 g (20 mmoles) de S-méthylcystéamine dans 40 cm³ de dichlorométhane est agitée à 0°C et additionnée goutte à goutte de 1,71 ml (20 mmoles) de chloro-2 éthyl-isocyanate, durant 15 min.

On laisse ensuite le mélange revenir à la température ambiante. La réaction suivie par CCM est complète en 4 h. La solution est alors évaporée à sec sous vide et la poudre obtenue est cristallisée dans un mélange de dichlorométhane-éther

(V/V). On recueille 3,2 g (81%) de S-méthyl N-[N-(chloro-2 éthyl)-N-carbamoyl] cystéamine.

CCM: (éluant: chloroforme-méthanol 8,5/1,5) Rf = 0,5 – Détection vapeurs d'iode. F = 80°C.

*Analyse* pour $C_6H_{13}SN_2OCl$ (196,5):

| | | | |
|---|---|---|---|
| Calculé: | C 36,64 | H 6,61 | N 14,24% |
| Trouvé: | C 36,41 | H 6,84 | N 14,30% |

*Spectre de RMN:* $H^1$ (DMSO d6) δ ppm:

N<u>H</u>: (m) centré à 6,22; 2 H; échangeable à $D_2O$

C<u>H</u>$_2$Cl: (m) centré à 3,56; 2 H

C<u>H</u>$_2$NH: (m) allant de 3,42 à 3,05; 4 H; ce multiplet est modifié après échange des N<u>H</u>

S–$CH_2$: (t) centré à 2,48; 2 H

C<u>H</u>$_3$: (s) 2,04; 3 H.

b) *Nitrosation de la S-méthyl N-[N-(chloro-2 éthyl) N-carbamoyl] cystéamine:*

Dans un bain d'eau glacée, on agite une solution de 1,47 g (7,5 mmoles) du composé obtenu en a) dans 15 $cm^3$ d'acide formique. La nitrosation est ensuite effectuée en additionnant durant 30 min 1,55 g (22,5 mmoles) de nitrite de sodium. On laisse revenir le mélange réactionnel à la température ambiante et l'agitation est maintenue 1 h. La solution jaune est ensuite additionnée de 70 $cm^3$ d'eau glacée, ce qui favorise le dépôt d'une gomme jaune dans le milieu. Cette gomme est extraite avec 2 × 50 $cm^3$ de dichlorométhane. Les phases organiques rassemblées sont lavées à l'eau distillée (2 × 10 $cm^3$) puis séchées sur sulfate de sodium et évaporées à sec sous vide. L'huile jaune recueillie sera purifiée sur colonne de silice (Kieselgel 60, 70-230 mesh) en utilisant un mélange éluant constitué de dichlorométhane et d'éther de pétrole (V/V). On obtient 1,43 g du mélange isomérique de S-méthyl N-[N-(chloro-2 éthyl)N-nitrosocarbamoyl] cystéamine et de S-méthyl N-nitroso-N[N-(chloro-2 éthyl)N-carbamoyl] cystéamine.

CCM (éluant = dichlorométhane) Rf = 0,4. Détection UV et vapeurs d'iode.

*Analyse* pour $C_6H_{12}SN_3O_2Cl$ (225,5):

| | | | |
|---|---|---|---|
| Calculé: | C 31,92 | H 5,32 | N 18,62% |
| Trouvé: | C 32,16 | H 5,59 | N 18,47% |

*Spectre de RMN* $H^1$ (DMSO d6) δ ppm:

N<u>H</u>: (m) centré à 8,91; 1 H; échangeable à $D_2O$

C<u>H</u>$_2$–N: (M9 centré à 4,0; 2 H
 |
 NO

C<u>H</u>$_2$–Cl; C<u>H</u>$_2$–NH: (m) allant de 3,85 à 3,36; 4 H; une partie de ce multiplet est modifiée après échange du N<u>H</u>.

S–C<u>H</u>$_2$: appartenant à la molécule nitrosée côté chlore: (t) centré à 2,67;

S–C<u>H</u>$_2$: appartenant à la molécule nitrosée côté soufre: (t) centré à 2,48.

Ces deux triplets de S–C<u>H</u>$_2$ s'intègrent comme deux protons. Le rapport des intégrations relatives de ces deux protons donne des proportions de 25% pour l'isomère nitrosé côté chlore et 75% pour l'isomère nitrosé côté soufre.

C<u>H</u>$_3$ appartenant à la molécule nitrosée côté chlore: (s) 2,10.

C<u>H</u>$_3$ appartenant à la molécule nirosée côté soufre: (s) 2,05.

Ces deux singulets C<u>H</u>$_3$ s'intègrent comme 3 protons dans les mêmes proportions que précédemment.

*Exemple 2:*

*N-[N-(chloro-2 éthyl)N-nitroso-carbamoyl] cystéamine* (schéma IV)

Une solution de 1,15 g (15 mmoles) de cystéamine (Fluka) fraîchement sublimée dans 90 $cm^3$ de benzène anhydre est agitée à température ambiante, sous courant d'azote, et additionnée par petites fractions de 4 g (14,6 mmoles) de N-(chloro-2 éthyl)-N-nitrosocarbamate de paranitrophénol (l'addition s'effectue durant 1 h).

La réaction suivie par CCM est complète en 12 h.

Le mélange réactionnel est alors évaporé à sec sous pression réduite. L'huile jaune résiduelle est ensuite chromatographiée sur colonne de silice sèche (Kieselgel 60, 70-230 mesh, Merck) avec le dichlorométhane comme éluant. On recueille 1,3 g de produit sous forme huileuse à température ambiante. Conservée au réfrigérateur et sous atmosphère d'azote, cette huile cristallise.

CCM (éluant: dichlorométhane) Rf = 0,30 – Détection UV et vapeurs d'iode.

*Analyse* pour $C_5H_{10}SN_3O_2Cl$ (211,5):

| | | | |
|---|---|---|---|
| Calculé: | C 28,36 | H 4,72 | N 19,85% |
| Trouvé: | C 28,29 | H 4,74 | N 19,72% |

*Spectre 1 R* (film) ν· $cm^{-1}$

(NH) 3380; ($CH_2$) 2980, 2940; (SH) 2580; (C=O) 1720; (amide) 1525; (N–NO) 1490.

*Spectre de RMN* $H^1$ ($CDCl_3$) δ ppm:

N<u>H</u>: (m) centré à 7,36; 1H; échangeable à $D_2O$

C<u>H</u>$_2$–N: (t) centré 'à 4,18; 2H
 |
 NO

NH–C<u>H</u>$_2$ et C<u>H</u>$_2$Cl: (m) allant de 3,80 à 3,42; 4 H; une partie de ce multiplet est modifiée après échange du N<u>H</u>

S–C<u>H</u>$_2$: (m) centré à 2,83; 2 H; ce multiplet est modifié après échange du SH

S<u>H</u>: (t) centré à 1,47; 1 H; échangeable à $D_2O$

L'oxydation du composé par l'iode en solution méthanolique a fourni avec un rendement quantitatif le dimère dinitrosé côté chlore qui est l'isomère minoritaire de la CNCC: le bis-N,N'-[N-(chloro-2 éthyl)-N-nitrosocarbamoyl]cystéamine, composé déjà décrit par deux autres voies de synthèse (brevet français N° 79 15672-J. Oiry, J.L. Imbach).

– «Nitrosoureas in Cancer Treatment», B. Serrou, P. Schein and J.L. Imbach, Eds., Elsevier/North Holland, New York, J.L. Imbach, J. Martinez, J. Oiry, Ch. Bourut, E. Chenu, R. Maral and G. Mathe, 1981, 123.

– J. Martinez, J. Oiry, J.L. Imbach et F. Winternitz, J. Med. Chem., 1982, *25*, 178.

*Exemple 3:*

*S-acétyl N-[N-(chloro-2 éthyl)N-nitrosocarbamoyl]cystéamine* (schéma IV)

Une solution de 1,060 g (5 mmoles) de N-[N-(chloro-2 éthyl)N-nitrosocarbamoyl]cystéamine obtenu à l'exemple 2 dans 10 $cm^3$ de pyridine fraîchement distillée est agitée à 0°C, sous courant d'azote et additionnée goutte à goutte de 0,71 $cm^3$ (10 mmoles) de chlorure d'acétyle fraîchement distillé. Le milieu réactionnel est maintenu à 0°C durant 1 h, puis on le laisse revenir à la température ambiante. La solution suivie par CCM est complète en 12 h. La solution est alors versée sur 100 $cm^3$ d'eau glacée, ce qui favorise le dépôt d'une gomme. Cette gomme est reprise par du dichlorométhane 2 × 50 $cm^3$. Les phases organiques rassemblées sont ensuite lavées à l'eau distillée (4 × 50 $cm^3$), séchées sur sulfate de sodium puis évaporées à sec sous vide. On recueille 1,250 g d'un produit huileux légèrement jaune. Cette huile sera purifiée par chromatographie sur colonne de silice (Kieselgel 60, 70-230 mesh, Merck) en utilisant le dichlorométhane comme éluant. Le dérivé S-acétylé est recueilli avec un rendement de 70%, soit 0,880 g.

CCM (éluant: dichlorométhane): Rf = 0,2 – Détection UV et vapeurs d'iode. Cristallise dans un mélange de dichlorométhane et d'éther de pétrole (2/8). F = 62-64°C.

*Analyse* pour $C_7H_{12}SN_3O_3O_3Cl$ (253,5):

| | | | |
|---|---|---|---|
| Calculé: | C 33,13 | H 4,73 | N 16,56% |
| Trouvé: | C 33,26 | H 4,70 | N 16,46% |

*Spectre I.R.* (KBr) ν $cm^{-1}$:
(NH) 3360; ($CH_2$) 3040, 2960; absence de bande (SH) à 2580; C=O) 1720, 1675; (amide) 1520; (N-NO) 1480.

*Spectre de RMN* $H^1$ ($CDCl_3$) δ ppm:
N$\underline{H}$: (m) centré à 7,27; 1 H; échangeable à $D_2O$
C$\underline{H}_2$–N: (t) centré à 4,19; 2 H
 |
 NO
NH–C$\underline{H}_2$: (m) centré à 3,70; 2H; ce multiplet est modifié après échange du N$\underline{H}$
C$\underline{H}_2$Cl: (t) centré à 3,52; 2H
S–C$\underline{H}_2$: (t) centré à 3,16; 2 H
C$\underline{H}_2$–C: (s) 2,38; 3H
 ‖
 O

*Exemple 4:*

*S-para-nitrobenzoyl N-[N-(chloro-2 éthyl)N-nitrosocarbamoyl]-cystéamine* (schéma IV)

La méthode utilisée est identique à celle mise en œuvre dans l'exemple 3. Dans ce cas le chlorure d'acétyle est remplacé par le chlorure de para-nitrobenzoyle.

La purification du composé se fait également par chromatographie sur colonne de silice avec le dichlorométhane comme éluant. Rdt = 70%.

CCM (éluant: dichlorométhane): Rf = 0,3 – Détection UV et vapeurs d'iode.

Cristallise dans un mélange de dichlorométhane et d'éther de pétrole (2/8) · F = 85-86°C.

*Analyse* pour $C_{12}H_{13}SN_4O_5Cl$ (360,5)

| | | | |
|---|---|---|---|
| Calculé: | C 39,94 | H 3,60 | N 15,53% |
| Trouvé: | C 39,83 | H 3,64 | N 15,49% |

*Spectre I.R.* (KBr) ν· $cm^{-1}$: (NH) 3400; (CH), ($CH_2$) 3120, 3080, 3050, 2980, 2940, 2870; absence de bande (SH) à 2580; (C=O) 1720, 1650; (amide) 1515; (N–NO) 1480.

*Spectre de RMN* $H^1$ ($CDCl_3$) δ ppm:
$\underline{H}$ aromatique: (m) centré à 8,20; 4H
N$\underline{H}$: (m) centré à 7,28; 1 H; échangeable à $D_2O$
C$\underline{H}_2$–N: (t) centré à 4,18; 2H
 |
 NO
NH–C$\underline{H}_2$: (m) centré à 3,83; 2 H; ce multiplet est modifié après échange du N$\underline{H}$
C$\underline{H}_2$Cl et S–C$\underline{H}_2$: (m) allant de 3,60 à 3,32; 4 H

*Exemple 5:*

*S-trityl-N-[N-(chloro-2 éthyl)N-nitrosocarbamoyl]-cystéamine*

La méthode utilisée est identique à celle utilisée dans l'exemple 2. Dans ce cas le produit de départ est la S-tritylcystéamine (préparée selon F.I. Carrol, H.M. Dickson et M.E. Wall, J. Org. Chem., 1965, *30*, 33) et le solvant utilisé est l'acétate d'éthyle. La purification du composé se fait par chromatographie sur colonne de silice en utilisant un mélange éluant constitué d'éther et d'éther de pétrole (1/9). Rdt = 68%.

CCM (éluant: acétate d'éthyle-éther de pétrole (1/4) RF = 0,5 – Détection UV et vapeurs d'iode. Cristallise dans un mélange d'éther et d'éther de pétrole (1/4) F = 112-113°C.

*Analyse* pour $C_{24}H_{24}SN_3O_2Cl$ (453,5):

| | | | |
|---|---|---|---|
| Calculé: | C 63,50 | H 5,29 | N 9,26% |
| Trouvé: | C 63,86 | H 5,27 | N 9,38% |

*Spectre I.R.* (KBr) ν· $cm^{-1}$: (NH) 3340; (CH), ($CH_2$) 3060, 3020, 2960, 2940; (C=O) 1720, 1690; (amide) 1520; (N–NO) 1490.

*Spectre de RMN* $H^1$ ($CDCl_3$) δ ppm:
$\underline{H}$ aromatiques: (m) centré à 7,30; 15 H
N$\underline{H}$: (m) centré à 6,90; 1 H; échangeable à $D_2O$
C$\underline{H}_2$–N: (t) centré à 4,10; 2 H
 |
 NO
C$\underline{H}_2$NH et C$\underline{H}_2$Cl: (m) allant de 3,60 à 3,04; 4 H; une partie de ce multiplet est modifiée après échange du N$\underline{H}$ S–C$\underline{H}_2$: (t) centré à 2,51; 2 H.

*Exemple 6:*

*Préparation de la S-méthyl N-nitroso-N-[-N-(chloro-2 éthyl) N-carbamoyl] cystéamine* (schéma II)

Le carbamate est préparé par action du chloroformiate de para nitro phényle (100 mM) sur la S-méthyl cystéamine (100 mM) dans la pyridine à température ambiante. Après 12 h de réaction on jette sur glace, filtre le précipité obtenu, lave à l'eau

glacée et sèche au dessiccateur sous vide sur $P_2O_5$.

Rdt = 60%.

La nitrosation est réalisée par le chlorure de nitrosyle dans la pyridine à −20°C selon les procédés décrits dans la littérature. Après 4 h de réaction à −20°C le milieu est hydrolysé par l'eau glacée, extrait par le chloroforme, la phase organique traitée par l'acide chlorhydrique est séchée sur Mg $SO_4$ puis distillée sous pression réduite. On obtient une huile utilisable pour la suite de la réaction. Rdt = 80%.

J. Martinez, J. Oiry, J.L. Imbach and F. Winternitz, J. Med. Chem., 25, 178-182, 1982.

A la solution de 20 mM de nitroso-carbamate dans 25 $cm^3$ de DMF, on ajoute goutte à goutte une solution de chloro-2-éthylamine (40 mM) libérée de son chlorhydrate par la triéthylamine.

On suit l'évolution de la réaction par CCM silice Merck Si 60 F 254, éluant $CHCl_3$. Après 30 min la réaction est terminée. On distille le solvant sous pression réduite et dépose le résidu sur colonne de silice. L'élution est menée par le chloroforme ou le dichlorométhane. Le produit du titre élué dans les fractions de tête est une huile. Rdt = 70%.

CCM Merck Si 60 F 254 éluant $CHCl_3$

Rf: 0,40

IR: Fenêtre (KBr) v· $cm^{-1}$: 3350 (NH), 2900 à 3000 (CH), 1710 (C) 1520 (NH−C=O),

‖
O

1480 (N−NO)

RMN $CDCl_3$ δ ppm

NO
|
[2,10 s (3) $CH_3$ S, 2,46 t (2) S $CH_2CH_2N$ ], [3,66 à 3,83 m (4) NH $CH_2CH_2$ Cl], [4,00 t (2) S

NO
|
$CH_2CH_2N$ ]

[7 à 7,60 m (1) NH $CH_2CH_2Cl$ échangeable].

*Exemple 7:*

*S-méthyl N-[N-(chloro-2 éthyl)N-nitrosocarbamoyl] cystéamine* (schéma III)

Les carbamate et nitroso-carbamate correspondants sont préparés selon les procédés de la littérature (idem précédemment) et adaptés au cas du 4-nitrophénol.

A la solution refroidie de 20 mM de nitroso-carbamate dans 30 $cm^3$ de THF on ajoute goutte à goutte sous agitation 20 mM de S-méthyl cystéamine. L'agitation est maintenue pendant 1 h à cette température.

L'avancement de la réaction est contrôlé par CCM (silice Merck Si 60 F 254 éluant $CHCl_3$). Après 1 h la réaction est terminée. Le milieu réactionnel est concentré sous vide et déposé sur colonne de silice et élué par $CH_2Cl_2$ ou $CHCl_3$ pour séparer le p-nitrophénol de la CNU (3).

Le produit élué avec les fractions de tête est une huile. Rdt = 90%

CCM Merck Si 60 F 254 éluant $CHCl_3$ Rf = 0,40

IR: KBr (v· $cm^{-1}$): 3350 (NH), 2900 à 3000 (CH), 1715 (C), 1520 (NH−C = O),

‖
O

1480 (N−NO)

RMN $CDCl_3$ δ ppm

[2,13 s (3) $CH_3S$], [2,73 t (2) S $CH_2CH_2NH$], [3,43 m (2) N−$CH_2CH_2Cl$], [3,70 m (2) S

|
NO

$CH_2CH_2NH$], [4,10 t (2) N $CH_2CH_2Cl$] [7 à

|
NO

7,50 m (1) S $CH_2CH_2NH$ échangeable]

La S-méthyl N-[N-(chloro-2-éthyl)N-nitrosocarbamoyl] cystéamine peut également être préparée de la façon suivante:

Une solution de 1,179 g de méthylthio-2 éthylamine ou S-méthylcystéamine à environ 90% (Fluka) dans 50 $cm^3$ de benzène anhydre est agitée à 20°C, sous courant d'azote, et additionnée par petites fractions de 2,98 g (10,9 mmoles) de N-(chloro-2 éthyl)N-nitrosocarbamate de paranitrophénol. (L'addition s'effectue durant 1 h et la réaction est exothermique, le mélange réactionnel est maintenu à 20°C dans un bain d'eau froide.) La réaction suivie par CCM est complète en 3 h. La solution est alors évaporée à sec sous pression réduite et l'huile résiduelle obtenue est chromatographiée sur colonne de silice (Kieselgel 60, 70-230 mesh, Merck) avec un mélange éluant constitué de dichlorométhane et d'éther de pétrole V-V. On recueille 1,85 g d'une huile jaune qui sera conservée sous azote au réfrigérateur.

CCM (éluant: dichlorométhane) Rf = 0,40 − Détection UV et vapeurs d'iode.

*Analyse* pour $C_6H_{12}SN_3O_2Cl$ (225,5):

| | | | |
|---|---|---|---|
| Calculé: | C 31,92 | H 5,32 | N 18,62% |
| Trouvé; | C 32,04 | H 5,27 | N 18,49% |

*Spectre I.R.* (film) v· $cm^{-1}$:

(NH) 3360; ($CH_2$) 2960, 2920, 2840; (C=O) 1720; (amide) 1520; (N−NO) 1490.

*Spectre de RMN* $H^1$ ($CDCl_3$) δ ppm:

NH: (m) centré à 7,43; 1 H; échangeable à $D_2O$

$CH_2$−N: (t) centré à 4,13; 2 H
|
NO

NH−$CH_2$: (m) centré à 3,67; 2 H; ce multiplet est modifié après échange du NH

$CH_2Cl$: (t) centré à 3,47; 2 H

S−$CH_2$: (t) centré à 2,73; 2 H

$CH_3$−S: (s) 2,10; 3 H

*Exemple 8:*

*Préparation des sulfoxydes du mélange de l'exemple 1* (schéma I)

O NO O
‖ | ‖
$CH_3$−S−$CH_2CH_2$−N − C−$NHCH_2CH_2Cl$ A1

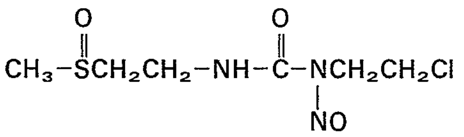
A2

A la solution dans l'acétone refroidie à 0°C de 15 mM de mélange isomérique on ajoute 15 $cm^3$ d'$H_2O_2$ à 110 v. On maintient 1 h sous agitation et contrôle l'avancement de la réaction par CCM.

Lorsque la réaction est terminée on dilue par $H_2O$ (30 $cm^3$) extrait par le chloroforme, sèche, distille le solvant sous pression réduite.

Le produit est déposé sur colonne de silice et chromatographié par un gradient d'éthanol dans le chloroforme de 0 à 2%.

On isole 7,5 mM de sulfoxyde (A1), 4 mM de mélange et 1 mM de sulfoxyde (A2).

F: A1 = 73°C. A2 = 107°C

CCM silice Merck Si 60 F 254 éluant $EtOH/CHCl_3$ 5%

Rf A1 = 0,30

Rf A2 = 0,25

IR: (KBr) v· $cm^{-1}$

A1 3220 (NH), 2900-3000 (CH), 1710 ($\underset{\underset{O}{\parallel}}{C}$),
1520 (NH$-\underset{\underset{O}{\parallel}}{C}$), 1480 (N$-$NO), 1030 (S=O)

A2 3220 (NH), 2900-3000 (CH), 1700 ($\underset{\underset{O}{\parallel}}{C}$),
1525 (NH$-\underset{\underset{O}{\parallel}}{C}$), 1480 (N$-$NO), 1045 (S=O)

RMN $CDCl_3$ δ ppm

A1 [2,60 s (3) $CH_3S$], [2,80 t (2) $S-CH_2CH_2-\underset{\underset{NO}{|}}{N}$] [3,60 à 3,90 m (4) $NH-CH_2CH_2Cl$], [4,20 t (2) $-SCH_2CH_2\underset{\underset{NO}{|}}{N}$] [7,60 à 8 m (1) $NHCH_2CH_2Cl$ échangeable]

A2 [2,60 s (3) $CH_3S$], [3,00 t (2) $SCH_2CH_2NH$] [3,40 t (2) $\overset{\overset{NO}{|}}{N}-CH_2CH_2-Cl$] [3,86 m (2) S $CH_2CH_2NH$] [4,10 t (2) $\underset{\underset{NO}{|}}{N}-CH_2CH_2Cl$] [7,40 à 7,60 m (1) $S-CH_2CH_2-NH$ échangeable]

Masse:

A1 m/z: 136 $[CH_3-SO-CH_2CH_2N=N-OH]^{+\cdot}$, m/z: 105 107 $[CO-N-CH_2CH_2Cl]^+$, m/z: 92 [m/z 136$-$(N$-$NO)$]^+$, m/z: 78 $[CH_3S\ OH-CH_2]^{+\cdot}$, m/z 64 $[CH_3S\ OH]^{+\cdot}$

A2 m/z: 134 $[CH_3SO\ CH_2CH_2NH-C\equiv O]^+$, m/z: 91 $[CH_3SOCH_2CH_2]^+$, m/z: 63,65 $[CH_2CH_2Cl]^+$, m/z 64: $[CH_3SOH]^+$.

*Exemple 9:*

*Préparation des sulfones du mélange de l'exemple 1*

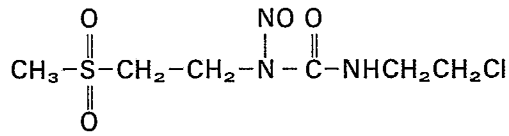
B1

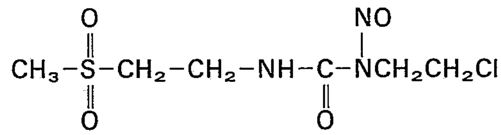
B2

Le protocole expérimental est le même que le précédent pour les sulfoxydes en utilisant l'acide formique au lieu de l'acétone.

Pour 15 mM de mélange isomérique on utilise 20 $cm^3$ d'acide formique et 20 $cm^3$ d'eau oxygénée à 110 v.

La séparation et l'extraction sont conduites de la même façon, l'élution est réalisée par un gradient éthanol dans le chloroforme de 0 à 1%.

On isole les mêmes quantités de composés purs et de mélange que dans l'expérimentation exemple 3.

F: B1: 72-76°C

B2: 94-97°C

CCM silice Merck Si 60 F 254 éluant EtOH. $CHCl_3$ 5%

RF B1: 0,55

RF B2: 0,50

IR: (KBr) v· $cm^{-1}$

B1 3300 (NH), 2900 à 3000 (CH), 1710 ($\underset{\underset{O}{\parallel}}{C}$),
1520 (NHCO), 1485 (N$-$NO),
1130 (O=S=O)

B2 3350 (NH), 2910 à 3000 (CH), 1715 ($\underset{\underset{O}{\parallel}}{C}$),
1525 (NH$-\underset{\underset{O}{\parallel}}{C}$) 1420 (N$-$NO),
1125 (O=S=O)

RMN: $CDCl_3$ δ ppm

B1 [2,93 s (3) $CH_3S$], [3,10 t (2) $-S\ CH_2CH_2NH$] [3,66 à 3,90 m (4) $\underset{\underset{NO}{|}}{HN}\ CH_2CH_2Cl$], [4,20 m (2) $-S-CH_2CH_2-\underset{\underset{NO}{|}}{N}$] [7,10 à 7,33 m (1) HN $CH_2CH_2Cl$ échangeable]

B2 [3,60 s $CH_3-S$], [3,26 à 3,66 m (4)
NO
|
S $CH_2CH_2NH$ et N $CH_2CH_2Cl$] [3,83 à 4,33 m (4)
NO
|
$S-CH_2CH_2NH$ et N $CH_2CH_2Cl$] [7,33 à 7,66 m (1) S $CH_2CH_2NH$ échangeable]

Masse:

B1 m/z: 152 $[CH_3SO_2CH_2CH_2\ N = N-OH]^{+\cdot}$, m/z 105.107 $[CO\ N\ CH_2CH_2Cl]^{+}$, m/z: 108 $[m/z\ 136-(N-NO)]^{+\cdot}$, m/z [94 $CH_3SO_2HCH_2]^{+\cdot}$, m/z: 80 $[CH_3SO_2H]^{+\cdot}$

B2 m/z: 150 $[CH_3SO_2CH_2CH_2NH-C{\equiv}O]^{+}$, m/z: 107 $[CH_3SO_2CH_2CH_2)^{+\cdot}$, m/z: 80 $[CH_2SO_2H]^{+\cdot}$, m/z: 63.65 $[CH_2CH_2Cl]^{+}$

*Exemple 10:*

En opérant comme décrit dans les exemples 8 et 9 à partir des composés issus des exemples 6 et 7 on obtient les sulfoxydes et les sulfones correspondants A1/A2/B1/B2 sous forme pure après chromatographie liquide/liquide.

Les Rf en CCM, les points de fusion, les spectres IR, RMN et de masse sont parfaitement analogues à ceux obtenus après séparation des mélanges isomères.

*Exemple 11:*

*Activité antitumorale des composés chloro-éthyl-nitroso-urées non oxydés selon l'invention*

L'activité oncostatique de ces dérivés nitrosés a été déterminée à l'ICIG de Villejuif (Service du Pr. Mathe), en suivant un protocole expérimental décrit: M. Hayat et Coll., Cancer Chemother. Pharmacol., 1979, *3*, 217-221, R. Maral et Coll., Eur. J. Med. Chem., soumis pour publication.

a) *Protocole expérimental*

Des souris $B_6D_2F_1$/OLA, mâles, sont inoculées avec $10^5$ cellules de la souche leucémique L1210 par voie intrapéritonéale au jour 0 et divisées en groupes. Le premier est le groupe témoin et les autres sont traités avec les drogues à différentes concentrations au jour 1,5 et 9. Les composés sont injectés en suspension dans l'huile d'olive. La mortalité des animaux est observée et l'autopsie indique si elle est due à la leucémie ou à l'action toxique des produits.

La dose létale 50% (DL50 aiguë) est déterminée en tenant compte de la mortalité survenue avant le huitième jour chez les animaux traités au jour J: 1. La médiane de survie des animaux traités (mT) et des animaux témoin (mC) a été établie et le test statistique non paramétrique de Wilcoxon a été utilisé. Les résultats sont considérés positifs si l'indice I ($I = \frac{mT}{mC} \times 100$) est supérieur à 125 et le test significatif à $p \leqslant 0{,}05$. La valeur $\infty$ indique que 50% ou plus des animaux sont survivants au jour +60.

b) *Résultats*

Plus l'intervalle des doses à efficacité maximale, ayant donc $I = \infty$, est important, plus le produit est doué d'une activité intéressante sur la leucémie L1210.

Nous constatons dans le tableau II que le mélange isomérique (8) constitué de S-méthyl N-[N-(chloro-2 éthyl)N-nitrosocarbamoyl] cystéamine (3) et de S-méthyl N-nitroso[N-chloro-2 éthyl)N-carbamoyl] cystéamine (4), bien que peu toxique, ne présente aucune activité. Ce mélange (3 + 4) étant constitué en majeure partie de NO côté soufre, l'orientation de ce travail a donc été axée vers des molécules nitrosées côté chlore. Trois molécules paraissent très intéressantes: en effet, la N-[N-(chloro-2 éthyl-N-nitrosocarbamoyl] cystéamine (1), la S-méthyl N-[N-(chloro-2 éthyl)N-nitrosocarbamoyl] cystéamine (3) et la S-acétyl N-[N-(chloro-2 éthyl) N-nitrosocarbamoyl] cystéamine (5), bien qu'ayant des DL50 allant de 19 à 25 mg/kg, présentent toutes des indices I à l'infini. De plus, ces trois composés sont susceptibles de conduire à 100% de survie à 60 jours, à différentes doses.

La S-trityl N-[N-(chloro-2 éthyl) N-nitrosocarbamoyl] cystéamine (9) n'est pas toxique puisque sa DL50 est supérieure à 625 mg/kg. Il est toutefois prématuré de juger son activité car les résultats cités ne sont que partiels.

| Composés | DL 50 mg/kg/i.p. | Doses mg/kg/i.p. | Jours | $I = \frac{T}{C} \times 100$ | % de survivants à 60 jours |
|---|---|---|---|---|---|
| (1) ICIG 2016 | 19 | 15<br>12,5<br>10<br>5<br>2<br>0,8<br>0,32 | + 1<br>1,5,9<br>1,5,9<br>1,5,9<br>1,5,9<br>1,5,9<br>1,5,9 | $\infty$<br>$\infty$<br>$\infty$<br>$\infty$<br>188<br>162<br>265 | 100<br>100<br>100<br>100 |

| Composés | DL 50 mg/kg/i.p. | Doses mg/kg/i.p. | Jours | $I = \frac{T}{C} \times 100$ | % de survivants à 60 jours |
|---|---|---|---|---|---|
| (3)<br>ICIG<br>2017 | 22 | 15<br>12,5<br>5<br>2<br>0,8<br>0,32 | 1,5,9<br>1,5,9<br>1,5,9<br>1,5,9<br>1,5,9<br>1,5,9 | 156<br>∞<br>∞<br>158<br>167<br>133 | <br>87<br>100 |
| (5)<br>ICIG<br>2018 | 25 | 20<br>15<br>12,5<br>10<br>5<br>4<br>1,6<br>0,64 | 1,5,9<br>1,5,9<br>1,5,9<br>1,5,9<br>1,5,9<br>1,5,9<br>1,5,9<br>1,5,9 | 211<br>490<br>∞<br>∞<br>217<br>158<br>117<br>100 | <br>25<br>80<br>100<br>14 |
| (8)<br>ICIG<br>1327 | 80 | 40<br>16 | 1,5<br>1,5 | 80 NS<br>100 NS | |
| (9)+<br>ICIG<br>2069 | > 625 | 625<br>250 | 1,5,9<br>1,5,9 | > 278<br>> 278 | |

\+ : résultats partiels
NS : non significatif

*Exemple 12:*

*Activité antitumorale des dérivés sulfoxydes ($A_1/A_2$) et sulfones ($B_1/B_2$) des composés selon l'invention*

Cette activité a également été étudiée chez la souris porteuse de la leucémie L 1210 greffée i.p. Cette tumeur expérimentale est la plus fréquemment utilisée dans le «screening» primaire des drogues cytostatiques.

Ces tests ont été menés parallèlement dans deux laboratoires: à l'Unité 71 de l'Institut National de la Santé et de la Recherche Médicale (INSERM), Clermont-Ferrand, et à l'Institut de Cancérologie et d'Immunogénétique (ICIG), Villejuif.

L'efficacité antitumorale des produits selon l'invention a été comparée à celle de la CNCC.

a) *Protocole expérimental*

Au jour J: 0, des souris mâles $B_6D_2F_1$ âgées de trois mois reçoivent par voie intrapéritonéale $10^5$ cellules de la souche leucémique L 1210. Les animaux sont répartis en lots de 8 à 10 animaux pour les groupes témoins et de 6 à 8 animaux pour les groupes traités. Aux jours J: 1, J: 5, J: 9, les animaux reçoivent par voie intrapéritonéale ou intraveineuse le traitement à différentes doses. La CNCC est administrée en suspension dans l'huile d'olive. Les produits selon l'invention sont administrés dans du sérum physiologique. La mortalité est enregistrée tous les jours. La médiane de survie des animaux traités et des animaux témoins a été déterminée. Les résultats sont considérés comme positifs lorsque l'index I =

$$\left(\frac{\text{médiane de survie des animaux traités}}{\text{médiane de survie des animaux témoins}} \times 100\right)$$

est supérieur à 125.

La valeur ∞ signifie que 50 à 100% des animaux sont survivants au jour J: 90.

b) *Résultats*

α – *Expérimentation réalisée à l'Unité 71 de l'INSERM*

L'activité cytostatique sur la leucémie L 1210 des produits selon l'invention a été évaluée chez des souris $B_6D_2F_1$/IFFA CREDO d'après le protocole précédemment décrit.

*Traitement par voie intrapéritonéale*

Ont été administrés par voie intrapéritonéale (i.p.) soit le mélange $A_1$ $A_2$ dans les proportions suivantes $A_1$ (70%) et $A_2$ (30%), soit le mélange $B_1$ $B_2$ dans les mêmes proportions, soit la CNCC aux doses suivantes: 10 mg/kg, 20 mg/kg, 30 mg/kg, 40 mg/kg et 50 mg/kg. Les résultats sont présentés sur le tableau II et appellent les commentaires suivants:

– très bonne efficacité du mélange $A_1$ $A_2$ aux doses de 20 mg/kg, 30 mg/kg et 40 mg/kg et du mélange $B_1$ $B_2$ aux doses de 20 mg/kg, 30 mg/kg, 40 mg/kg et 50 mg/kg. Ces mélanges présentent

un intervalle de dose à efficacité maximale (IDEM) comparable et même supérieur pour le mélange $B_1$ $B_2$ à celui de la CNCC. De plus, le nombre de survivants à 90 jours est plus grand chez les animaux ayant reçu les produits selon l'invention par rapport aux animaux ayant reçu la CNCC.

*Traitement par voie intraveineuse*

Ont été administrés par voie intraveineuse (i.v.) dans la veine caudale, les mélanges $A_1$ $A_2$ et $B_1$ $B_2$ dans les proportions et aux doses utilisées dans a). La comparaison avec la CNCC pour cette voie d'administration est impossible en raison de l'insolubilité de ce composé dans les solvants autorisant l'administration par voie intraveineuse. Les résultats sont présentés sur le tableau III.

Le mélange $A_1$ $A_2$ est très actif aux doses de 30 mg/kg et 40 mg/kg. L'IDEM du mélange $B_1$ $B_2$ est supérieur (30 mg/kg, 40 mg/kg et 50 mg/kg).

β – *Expérimentation réalisée à l'ICIG*

L'activité cytostatique sur la leucémie L 1210 de $A_1$ et $A_2$ a été évaluée à l'ICIG chez des souris $B_6D_2F_1$/OLA.

Le traitement des animaux par le mélange $A_1$ $A_2$ dans les proportions de l'$A_1$ (70%) et $A_2$ (30%) donne des résultats très comparables à ceux obtenus à l'Unité 71 de l'INSERM (tableau III).

Il a en outre été procédé à l'ICIG à une étude de l'activité antitumorale sur la leucémie L 1210 de chaque isomère $A_1$ et $A_2$ administré séparément par voie intraveineuse. Les résultats sont rassemblés sur le tableau IV. On constate une faible activité de $A_1$ et une forte activité de $A_2$ avec présence d'un IDEM et l'obtention d'un pourcentage élevé de souris survivantes au jour J : 100. La comparaison des doses actives à celles de la CNCC est impossible pour les raisons d'insolubilité de la CNCC énoncées précédemment.

*Tableau III*

Activité antitumorale sur la leucémie L 1210 greffée i.p. chez la souris des mélanges $A_1$ $A_2$ et $B_1$ $B_2$

| Voie d'administration | Proportions du mélange | Dose mg/kg T/C × 100 | | | | |
|---|---|---|---|---|---|---|
| | | 10 | 20 | 30 | 40 | 50 |
| i.p. | $A_1$ 70% $A_2$ 30% | 300(2/6) | ∞(6/6) | ∞(6/6) | ∞(5/6) | 295(0/6) |
| | $B_1$ 70% $B_2$ 30% | 190(0/6) | ∞(5/6) | ∞(6/6) | ∞(6/6) | ∞(5/6) |
| | CNCC | 250(0/6) | ∞(6/6) | ∞(6/6) | ∞(3/6) | 125(0/6) |
| i.v. | $A_1$ 70% $A_2$ 30% | 170(0/6) | 270(0/6) | ∞(6/6) | ∞(6/6) | ∞(3/6) |
| | $B_1$ 70% $B_2$ 30% | 190(0/6) | 230(0/6) | ∞(6/6) | ∞(6/6) | ∞(5/6) |

Traitement $J_1$, $J_5$, $J_9$.

$$T/C = \frac{\text{Médiane de survie des animaux traités}}{\text{Médiane de survie des animaux témoins}} \times 100$$

∞ = 50 à 100% des animaux survivants à 90 jours

( ) = Nombre de survivants à 90 jours

*Tableau IV*

Activité antitumorale sur la leucémie L 1210 greffée i.p. chez la souris de $A_1$ et $A_2$ injectés séparément

| Voie d'administration | Produit | Dose mg/kg T/C × 100 | | | | | | |
|---|---|---|---|---|---|---|---|---|
| i.v. | | 5 | 10 | 20 | 30 | 40 | 50 | 60 |
| | $A_1$ | 100 | 100 | 133 | 133 | 158 | 158 | Toxique |
| | | 1 | 5 | 10 | 20 | 30 | 40 | — |
| | $A_2$ | 100 | ∞ | ∞ | 63 | 63 | 63 | |

*Exemple 13:*

*Essais de pharmacocinétique*

La cinétique d'absorption et d'épuration plasmatique des produits selon l'invention $A_1$ et $A_2$ a été étudiée chez différentes espèces animales après administration orale. Pour pouvoir établir une comparaison avec la CNCC, un mélange $A_1$ $A_2$ dans les proportions 70%–30% a été utilisé. Le mélange $A_1$ $A_2$ et la CNCC ont été administrés à doses équivalentes et les concentrations plasmatiques en fonction du temps de $A_1$ et $A_2$ ont été mesurées.

a) *Protocole expérimental*

Le mélange $A_1$ $A_2$ dissous dans l'eau distillée est administré par sonde gastrique à la dose de 574 mg/m². La CNCC en suspension dans l'huile d'olive est également administrée par sonde gastrique à la dose de 500 mg/m².

α – *Préparation des échantillons*

Le sang prélevé par ponction cardiaque est rapidement centrifugé à 4°C. Le plasma (1 ml) est déposé sur une cartouche d'Extrelut®, l'extraction est effectuée avec 6 ml de dichlorométhane. Après évaporation, l'extrait sec est repris dans 150 µl de dichlorométhane pour l'analyse chromatographique.

β – *Analyse chromatographique*

L'analyse des extraits plasmatiques pour la mesure des concentrations de $A_1$ et $A_2$ a été effectuée par chromatographie liquide haute performance selon la technologie suivante:

- Colonne (40 cm × 0,46 cm) Si 100 10 µm (Merck).
- Détecteur: Spectrophotomètre Pye Unicam (Philips), λ 250 nm.
- Débit: 2 ml/min.
- Elution réalisée par un gradient linéaire allant de dichlorométhane 99,6% - Ethanol 0,4% jusqu'à dichlorométhane 95% - Ethanol 5% en 15 min.
- Temps de rétention: $A_1$ 18,7 min, $A_2$ 20 min.

b) *Résultats*

Sur le tableau V sont rapportées les concentrations plasmatiques de $A_1$ et $A_2$ après administration orale à la souris, au rat et au lapin soit du mélange $A_1$ $A_2$, soit de la CNCC.

Quelle que soit l'espèce animale, les concentrations de $A_1$ et $A_2$ sont beaucoup plus élevées dans le cas de l'administration des produits selon l'invention $A_1$ et $A_2$ que dans le cas de l'administration de la CNCC. La mesure des aires sous les courbes confirme ces résultats.

Ces données pharmacocinétiques préliminaires tendent à montrer que la concentration plasmatique du composé actif $A_2$ (cf. Exemple 12) est nettement supérieure dans le cas de l'administration de $A_1$ $A_2$ que dans le cas de l'administration de la CNCC.

Il existe donc une biodisponibilité plus grande des produits selon l'invention $A_1$ et $A_2$ comparés à la CNCC.

*Tableau V*

Comparaison des concentrations plasmatiques de $A_1$ et $A_2$ après administration P,O, du mélange $A_1$ et $A_2$ et de CNCC à dose équivalente

| Produits administrés | Animal | nmoles/ml | 5 min | 15 min | 30 min | 45min | 1 h | 1 h 30 | 2 h | 3 h | 4 h 30 |
|---|---|---|---|---|---|---|---|---|---|---|---|
| $A_1$ $A_2$ | Souris (n=10) | $A_1$ | 208,0 | 206,0 | 177,0 | 124,0 | 88,0 | 35,4 | 23,3 | 10,1 | 4,3 |
| | | $A_2$ | 83,0 | 81,5 | 66,3 | 43,7 | 29,9 | 10,8 | 6,3 | 2,9 | 0,9 |
| CNCC | | $A_1$ | 13,7 | 34,2 | 71,4 | 32,8 | 37,1 | 26,2 | 20,2 | 11,0 | 6,5 |
| | | $A_2$ | 5,3 | 13,2 | 35,7 | 16,5 | 19,0 | 12,7 | 9,9 | 6,7 | 3,5 |
| $A_1$ $A_2$ | Rats (n=5) | $A_1$ | 22,3 | 42,4 | 52,9 | 33,9 | 32,9 | 29,2 | 15,7 | 7,3 | — |
| | | $A_2$ | 8,5 | 17,7 | 22,5 | 11,4 | 14,2 | 12,9 | 7,8 | 2,9 | — |
| CNCC | | $A_1$ | 2,8 | 8,0 | 12,0 | 14,8 | 18,4 | 20,3 | 12,7 | 11,1 | 3,9 |
| | | $A_2$ | 0,9 | 3,2 | 5,4 | 7,2 | 8,1 | 11,4 | 6,4 | 6,5 | 2,2 |
| $A_1$ $A_2$ | Lapins (n=5) | $A_1$ | 26,7 | 28,5 | 41,0 | 39,2 | 39,4 | 27,0 | 19,4 | 10,6 | 3,8 |
| | | $A_2$ | 11,0 | 11,7 | 16,5 | 14,2 | 14,2 | 9,2 | 6,3 | 2,9 | 0,7 |
| CNCC | | $A_1$ | | 5,0 | 9,4 | 10,6 | 10,0 | 8,6 | 7,4 | 4,7 | 2,8 |
| | | $A_2$ | | 1,8 | 5,0 | 6,0 | 5,5 | 4,2 | 3,5 | 2,0 | 1,0 |

**Revendications**

1. Composé de formule générale I:

$$R-S(O)_n-(CH_2)_2-\underset{X}{N}-\underset{O}{\overset{}{C}}-\underset{Y}{N}-(CH_2)_2-Cl$$

dans laquelle:

– X et Y sont définis individuellement par H ou –NO, l'un au moins étant un groupement –NO,

– n est égal à 0, 1 ou 2,

– R est l'hydrogène, un radical aliphatique, cycloaliphatique, cycloaliphatique-alkyl, aryle ou

aralcoyle, ou bien encore un groupement $R_1-C(=O)-$ dans lequel $R_1$ est un radical alkyle inférieur ou aryle,
l'ensemble de ces radicaux étant éventuellement substitué.

2. Composés de formule I:

$$R-S(O)_n-(CH_2)_2-N(X)-C(=O)-N(Y)-(CH_2)_2-Cl$$

dans laquelle:
– X, Y et n possèdent les mêmes significations qu'indiqué à la revendication 1,
– R étant l'hydrogène, un radical alkyle, aralcoyle ou bien un groupement $R_1-C(=O)-$ dans lequel $R_1$ est un radical alkyle, phényle ou phényle substitué.

3. Composé de formule:

$$H-S(O)_n-(CH_2)_2-NH-C(=O)-N(NO)-(CH_2)_2-Cl$$

4. Composé de formule:

$$CH_3S(O)_n-(CH_2)_2-N(NO)-C(=O)-NH-(CH_2)_2-Cl$$

ou

$$CH_3-S(O)_n-(CH_2)_2-NH-C(=O)-N(NO)-(CH_2)_2-Cl$$

5. Mélange isomérique des composés selon la revendication 4.

6. Composé selon l'une des revendications 1 ou 2, caractérisé en ce que R est $(C_6H_5)_3-C-$.

7. Composé selon l'une des revendications 1 ou 2, caractérisé en ce-que R est le groupement $R_1-C(=O)-$ dans lequel $R_1$ est:

$-CH_3$ ou 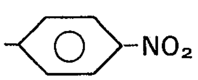

8. Procédé de préparation des composés selon la revendication 1, dans lesquels n est égal à 0 et R est H, un radical aliphatique, cycloaliphatique, cycloaliphatique-alkyl, aryle ou aralcoyle, caractérisé en ce qu'on effectue une nitrosation du composé correspondant de formule:

$$R-S-(CH_2)_2-NH-C(=O)-NH-(CH_2)_2-Cl$$

par action d'un nitrite de métal alcalin ou alcalino-terreux, en particulier $NO_2Na$, en présence d'acide organique tel que l'acide formique.

9. Procédé de préparation des composés selon la revendication 1 dans lesquels n est égal à 0 et R est H, un radical aliphatique, cycloaliphatique, cycloaliphatique-alkyl, aryle ou aralcoyle, caractérisé en ce qu'on fait réagir:

$$H_2N-(CH_2)_2-Cl$$

sur $X_1-O-C(=O)-N(NO)-(CH_2)_2-S-R$

dans lequel $X_1$ est un groupe activant pour obtenir le dérivé nitrosé côté soufre ou:

$$R-S-(CH_2)_2-NH_2$$

sur $X_2-O-C(=O)-N(NO)-(CH_2)_2-Cl$

dans lequel $X_2$ est un groupe activant pour obtenir le dérivé nitrosé côté chlore.

10. Procédé de préparation des composés selon la revendication 1 dans lesquels R est un groupement $R_1-C(=O)-$ et n est égal à 0, caractérisé en ce qu'on effectue une thioestérification d'un composé de formule:

$$H-S-(CH_2)_2-N(X)-C(=O)-N(Y)-(CH_2)_2-Cl$$

par un dérivé réactif de l'acide correspondant

$$R_1-C(=O)-OH.$$

11. Procédé de préparation des composés de formule I dans lesquels n = 1 ou n = 2, caractérisé en ce qu'on effectue une oxydation ménagée ou complète des composés de formule I correspondants préparés selon l'une des revendications 8 à 10.

12. Oxydation ménagée selon la revendication 11, caractérisée en ce qu'on soumet les composés de formule I préparés selon l'une des revendications 8 à 10, à l'action d'un peroxyde, tel que le peroxyde d'hydrogène, en présence d'acétone, de manière à obtenir les sulfoxydes correspondants (n = 1).

13. Oxydation complète selon la revendication 11, caractérisée en ce qu'on soumet les composés de formule I préparés selon l'une des revendications 8 à 10, à l'action d'un peroxyde, tel que le peroxyde d'hydrogène, en présence d'acide formique de manière à obtenir les sulfones correspondants (n = 2).

14. Composition pharmaceutique comportant à titre de principe actif au moins un des composés selon la revendication 1.

15. Composition pharmaceutique selon la revendication 14, utile en chimiothérapie anticancéreuse.

## Patentansprüche

1. Verbindung der allgemeinen Formel (I):

$$R-S(O)_n-(CH_2)_2-\underset{X}{\underset{|}{N}}-\underset{O}{\underset{\|}{C}}-\underset{Y}{\underset{|}{N}}-(CH_2)_2-Cl$$

worin bedeuten:
– X und Y einzeln jeweils H oder –NO, wobei mindestens einer davon eine –NO–Gruppe darstellt,
– n die Zahl 0, 1 oder 2,
– R Wasserstoff, einen aliphatischen, cycloaliphatischen, cycloaliphatischen Alkyl-, Aryl- oder Aralkylrest oder auch eine Gruppe $R_1-\underset{O}{\underset{\|}{C}}-$, worin $R_1$ einen niederen Alkyl- oder Arylrest darstellt, wobei die Gesamtheit dieser Reste ggf. substituiert ist.

2. Verbindung der Formel (I):

$$R-S(O)_n-(CH_2)_2-\underset{X}{\underset{|}{N}}-\underset{O}{\underset{\|}{C}}-\underset{Y}{\underset{|}{N}}-(CH_2)_2-Cl$$

worin bedeuten:
– X, Y und n die gleichen Bedeutungen wie im Anspruch 1 angegeben,
– R Wasserstoff, einen Alkylrest, einen Aralkylrest oder eine Gruppe $R_1-\underset{O}{\underset{\|}{C}}-$, worin $R_1$ einen Alkyl-, Phenyl- oder substituierten Phenylrest darstellt.

3. Verbindung der Formel:

$$H-S(O)_n-(CH_2)_2-NH-\underset{O}{\underset{\|}{C}}-\underset{NO}{\underset{|}{N}}-(CH_2)_2-Cl$$

4. Verbindung der Formel:

$$CH_3S(O)_n-(CH_2)_2-\underset{NO}{\underset{|}{N}}-\underset{O}{\underset{\|}{C}}-NH-(CH_2)_2-Cl$$

oder

$$CH_3-S(O)_n-(CH_2)_2-NH-\underset{O}{\underset{\|}{C}}-\underset{NO}{\underset{|}{N}}-(CH_2)_2-Cl$$

5. Isomerengemisch der Verbindung nach Anspruch 4.

6. Verbindung nach Anspruch 1 oder 2, dadurch gekennzeichnet, dass R $(C_6H_5)_3-C-$ darstellt.

7. Verbindung nach Anspruch 1 oder 2, dadurch gekennzeichnet, dass R die Gruppe $R_1-\underset{O}{\underset{\|}{C}}-$ darstellt, wobei $R_1$

$-CH_3$ oder 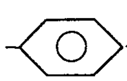 $-NO_2$ bedeutet.

8. Verfahren zur Herstellung der Verbindungen nach Anspruch 1, in denen n die Zahl 0 und R H, einen aliphatischen, cycloaliphatischen, cycloaliphatischen Alkyl-, Aryl- oder Aralkylrest bedeutet, dadurch gekennzeichnet, dass man eine Nitrosierung der Verbindung der Formel:

$$R-S-(CH_2)_2-NH-\underset{O}{\underset{\|}{C}}-NH-(CH_2)_2-Cl$$

mittels eines Alkalimetall- oder Erdalkalimetallnitrits, insbesondere mittels $NaNO_2$, in Gegenwart einer organischen Säure, wie Ameisensäure, bewirkt.

9. Verfahren zur Herstellung der Verbindungen nach Anspruch 1, in denen n die Zahl 0 und R H, einen aliphatischen, cycloaliphatischen, einen cycloaliphatischen Alkyl-, Aryl- oder Aralkylrest bedeuten, dadurch gekennzeichnet, dass man

$$H_2N-(CH_2)_2-Cl$$

mit $X_1-O-\underset{O}{\underset{\|}{C}}-\underset{NO}{\underset{|}{N}}-(CH_2)_2-S-R$

worin $X_1$ eine aktivierende Gruppe darstellt, reagieren lässt zur Herstellung des auf der Schwefelseite nitrosierten Derivats oder dass man

$$R-S-(CH_2)_2-NH_2$$

mit $X_2-O-\underset{O}{\underset{\|}{C}}-\underset{NO}{\underset{|}{N}}-(CH_2)_2-Cl$

worin $X_2$ eine aktivierende Gruppe darstellt, reagieren lässt zur Herstellung des auf der Chlorseite nitrosierten Derivats.

10. Verfahren zur Herstellung der Verbindungen nach Anspruch 1, in denen R eine Gruppe $R_1-\underset{O}{\underset{\|}{C}}-$ und n die Zahl 0 bedeuten, dadurch gekennzeichnet, dass man eine Verbindung der Formel:

$$H-S-(CH_2)_2-\underset{X}{\underset{|}{N}}-\underset{O}{\underset{\|}{C}}-\underset{Y}{\underset{|}{N}}-(CH_2)_2-Cl$$

einer Thioveresterung unterwirft unter Verwendung eines reaktionsfähigen Derivats der entsprechenden Säure der Formel $R_1-\underset{O}{\underset{\|}{C}}-OH$.

11. Verfahren zur Herstellung der Verbindung der Formel (I), in der n die Zahl 1 oder 2 bedeutet, dadurch gekennzeichnet, dass man eine schonende oder vollständige Oxidation der entsprechenden Verbindungen der Formel (I), die nach einem der Ansprüche 8 bis 10 hergestellt worden sind, bewirkt.

12. Schonende Oxidation nach Anspruch 11, dadurch gekennzeichnet, dass man die Verbindungen der Formel (I), die nach einem der Ansprüche 8 bis 10 hergestellt worden sind, der Einwirkung eines Peroxids, wie Wasserstoffperoxid, in Gegenwart von Aceton unterwirft, so dass man die entsprechenden Sulfoxide (n = 1) erhält.

13. Vollständige Oxidation nach Anspruch 11, dadurch gekennzeichnet, dass man die Verbindungen der Formel (I), die nach einem der Ansprüche 8 bis 10 hergestellt worden sind, der Wirkung eines Peroxids, wie Wasserstoffperoxid, in Gegenwart von Ameisensäure unterwirft, so dass man die entsprechenden Sulfone (n = 2) erhält.

14. Pharmazeutische Zusammensetzung, die als Wirkstoff mindestens eine der Verbindungen nach Anspruch 1 enthält.

15. Pharmazeutische Zusammensetzung nach Anspruch 14, die in der Antikrebs-Chemotherapie brauchbar ist.

## Claims

1. A compound of the general formula I:

$R-S(O)_n-(CH_2)_2-N-C-N-(CH_2)_2-Cl$
| || |
X O Y

in which:

– X and Y are individually defined as H or –NO, at least one being an –NO group,

– n is equal to 0, 1 or 2, and

– R is hydrogen, an aliphatic, cycloaliphatic, cycloaliphatic-alkyl, aryl or aralkyl radical or alternatively a group $R_1-C-$, in which $R_1$ is a lower
||
O
alkyl or aryl radical,
all these radicals being optionally substituted.

2. A compound of the formula I:

$R-S(O)_n-(CH_2)_2-N-C-N-(CH_2)_2-Cl$
| || |
X O Y

in which:

– X, Y and n possess the same meanings as indicated in Claim 1,

– R being hydrogen, an alkyl or aralkyl radical or alternatively a group $R_1-C-$, in which $R_1$ is an
||
O
alkyl, phenyl or substituted phenyl radical.

3. A compound of the formula:

$H-S(O)_n-(CH_2)_2-NH-C-N-(CH_2)_2-Cl$
|| |
O NO

4. A compound of the formula:

$CH_3S(O)_n-(CH_2)_2-N-C-NH-(CH_2)_2-Cl$
| ||
NO O

or

$CH_3-S(O)_n-(CH_2)_2-NH-C-N-(CH_2)_2-Cl$
|| |
O NO

5. An isomeric mixture of the compounds as claimed in Claim 4.

6. A compound as claimed in one of Claims 1 or 2, wherein R is $(C_6H_5)_3-C-$.

7. A compound as claimed in one of Claims 1 or 2, wherein R is the group $R_1-C-$, in which $R_1$ it:
||
O

$-CH_3$ or – 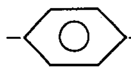 $-NO_2$

8. A process for the preparation of the compounds as claimed in Claim 1 in which n is equal to 0 and R is H or an aliphatic, cycloalphatic, cycloaliphatic-alkyl, aryl or aralkyl radical, which comprises nitrosating the corresponding compound of the formula:

$R-S-(CH_2)_2-NH-C-NH-(CH_2)_2-Cl$
||
O

by reaction with an alkali metal nitrite or alkaline earth metal nitrite, in particular $NO_2Na$, in the presence of an organic acid such as formic acid.

9. A process for the preparation of the compounds as claimed in Claim 1 in which n is equal to 0 and R is H or an aliphatic, cycloaliphatic, cycloaliphatic-alkyl, aryl or aralkyl radical, which comprises reacting $H_2N-(CH_2)_2-Cl$ with

$H_2N-(CH_2)_2-Cl$

with $X_1-O-C-N-(CH_2)_2-S-R$
|| |
O NO

in which $X_1$ is an activating group, to give the derivative nitrosated nearer the sulfur, or reacting

$R-S-(CH_2)_2-NH_2$

with $X_2-O-C-N-(CH_2)_2-Cl$
|| |
O NO

in which $X_2$ is an activating group, to give the derivative nitrosated nearer the chlorine.

10. A process for the preparation of the compounds as claimed in Claim 1 in which R is a group $R_1-C-$ and n is equal to 0, which comprises carry-
||
O
ing out the thioesterification of a compound of the formula:

$H-S-(CH_2)_2-N-C-N-(CH_2)_2-Cl$
| || |
X O Y

with a reactive derivative of the corresponding acid $R_1-\underset{\underset{O}{\|}}{C}-OH$

11. A process for the preparation of the compounds of the formula I in which n = 1 or n = 2, which comprises carrying out the controlled or complete oxidation of the corresponding compounds of the formula I, prepared as claimed in one of Claims 8 to 10.

12. Controlled oxidation as claimed in Claim 11, which comprises reacting the compounds of the formula I, prepared as claimed in one of Claims 8 to 10, with a peroxide such as hydrogen peroxide, in the presence of acetone, to give the corresponding sulfoxides (n = 1).

13. Complete oxidation as claimed in Claim 11, which comprises reacting the compounds of the formula I, prepared as claimed in one of Claims 8 to 10, with a peroxide such as hydrogen peroxide, in the presence of formic acid, to give the corresponding sulfones (N = 2).

14. A pharmaceutical composition containing, as the active principle, at least one of the compounds as claimed in Claim 1.

15. A pharmaceutical composition as claimed in Claim 14, which is useful in anticancer chemotherapy.